(19) Europäisches Patentamt
European Patent Office
Office européen des brevets

(11) **EP 3 960 189 A1**

(12) **EUROPEAN PATENT APPLICATION**

(43) Date of publication:
**02.03.2022 Bulletin 2022/09**

(21) Application number: **20205703.0**

(22) Date of filing: **04.11.2020**

(51) International Patent Classification (IPC):
*A61K 35/618* (2015.01)   *A61K 35/64* (2015.01)
*A61K 36/07* (2006.01)   *A61P 31/12* (2006.01)
*A61P 31/14* (2006.01)

(52) Cooperative Patent Classification (CPC):
(C-Sets available)
**A61P 31/14; A61K 35/618; A61K 35/64;
A61K 36/07; A61P 31/12**   (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.08.2020 KR 20200106422**

(71) Applicant: **Apexel Co., Ltd.
Gyeongsangbuk-do 37510 (KR)**

(72) Inventor: **KIM, Cheong Ja
37510 Gyeongsangbuk-do (KR)**

(74) Representative: **Lambsdorff & Lange
Patentanwälte
Partnerschaft mbB
Grillparzerstraße 12A
81675 München (DE)**

(54) **ANTIVIRAL COMPOSITION HAVING ANTIVIRAL ACTIVITY AGAINST CORONAVIRUS AND VARIANT VIRUSES THEREOF**

(57)    Disclosed is an antiviral composition having excellent antiviral activity against coronavirus and variant viruses thereof, more particularly, an antiviral composition containing an oyster shell powder; an *Inonotus obliquus* powder; and a silkworm powder or a *Gryllus bimaculatus* powder; as an active ingredients. The antiviral composition has activity of enhancing immunity that acts as an *in-vivo* defense against infection with harmful substances such as bacteria and viruses, and has activity of effectively inhibiting the proliferation of coronavirus, and thus has useful effects as a drug or health functional food for the prevention, amelioration or treatment of coronaviral infection.

Fig.1

(52) Cooperative Patent Classification (CPC): (Cont.)

C-Sets
**A61K 35/618, A61K 2300/00;**
**A61K 35/64, A61K 2300/00;**
**A61K 36/07, A61K 2300/00**

**Description**

BACKGROUND OF THE INVENTION

Field of the Invention

The present invention relates to an antiviral composition having excellent antiviral activity against coronavirus and variant viruses thereof.

Description of the Related Art

Corona virus is an enveloped single-stranded positive RNA virus, has a genome size of 25-32 kb, and is one of the larger RNA viruses known to date. Corona virus a specific flame- or crown-shaped structure in which spike proteins, which are club-shaped protrusions, are embedded in the envelope.

Coronavirus, which infects humans, is known to cause colds, upper respiratory tract infections, respiratory diseases, diarrhea in children, and other intestinal diseases, and has been recognized as a virus that mainly infects animals such as dogs, pigs and cattle.

Meanwhile, when the bacteria causative of severe acute respiratory syndrome (SARS) became known as a novel (variant) coronavirus, coronavirus began to attract great attention. Severe acute respiratory syndrome (SARS) was highly prevalent in China between November 2002 and June 2003, causing 8,096 infections and 774 deaths (Cinatl, J., et al., 2003; Rota., PA, et al., 2003).

Moreover, recently, coronavirus disease 2019 (COVID-19) has emerged worldwide as a novel respiratory infectious disease caused by SARS-CoV-2 (severe acute respiratory syndrome coronavirus 2). Since the first outbreak in Wuhan, China in December 2019, COVID-19 has spread worldwide, and the number of confirmed cases in South Korea has increased rapidly, resulting in 8,086 patients and 72 (0.9%) deaths as of March 14, 2020. The number of confirmed cases is increasing rapidly all over the world, including Korea, but vaccines or therapeutic agents therefor have not been developed at present and thus COVID-19 has emerged as a serious worldwide health problem.

Based on analysis of data on more than 70,000 patients in China, it has been reported that about 15% of patients suffer from severe pneumonia, about 5% of patients need intensive care, and about half of patients who receive intensive care die. As such, the main symptoms of infection with Covid-19 include feelings of lethargy, high fever of 37.5 degrees or higher, cough, sore throat, phlegm, muscle pain, headache, dyspnoea and pneumonia, and respiratory failure due to lung damage, leading in severe cases to death. Therefore, there is an urgent need for development of an agent for prevention, amelioration or treatment of coronavirus infection-19.

Prior Art

Patent Document

(Patent Document 1) Korean Patent No. 10-1976528
(Patent Document 2) Korean Patent No. 10-2109196

SUMMARY OF THE INVENTION

Accordingly, the present inventors found that the composition containing a mixed powder obtained by ultra-fine granulation of naturally derived ingredients, namely, an oyster shell; *Inonotus obliquus;* and silkworm or *Gryllus bimaculatus;* has excellent antiviral activity against coronavirus. Based on this finding, the present invention has been completed.

Accordingly, it is an object of the present invention to provide an antiviral composition containing an oyster shell powder; an *Inonotus obliquus* powder; and a silkworm powder or a *Gryllus bimaculatus* powder; as active ingredients.

In accordance with the present invention, the above and other objects can be accomplished by the provision of an antiviral composition containing an oyster shell powder; an *Inonotus obliquus* powder;and a silkworm powder or a *Gryllus bimaculatus* powder; as an active ingredients.

In one embodiment of the present invention, the oyster shell powder may be present in an amount of 40 to 60 parts by weight, the *Inonotus obliquus* powder may be present in an amount of 10 to 30 parts by weight, and the silkworm powder or the *Gryllus bimaculatus* powder may be present in an amount of 10 to 30 parts by weight.

In one embodiment of the present invention, the antiviral composition may further contain a red ginseng powder,

wherein the red ginseng powder is present in an amount of 10 to 20 parts by weight.

In one embodiment of the present invention, the powder may be an ultrafine powder having a particle size of 50 nm to 5 $\mu$m.

In one embodiment of the present invention, the antiviral composition may have antiviral activity against coronavirus.

BRIEF DESCRIPTION OF THE DRAWINGS

The above and other objects, features and other advantages of the present invention will be more clearly understood from the following detailed description taken in conjunction with the accompanying drawings, in which:

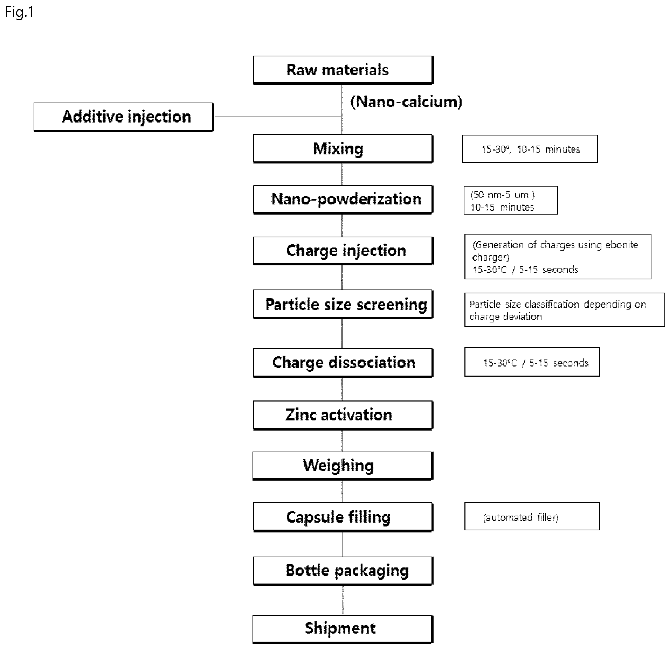

FIG. 1 is a schematic diagram showing a process for preparing an antiviral composition according to the present invention.

DETAILED DESCRIPTION OF THE INVENTION

The present invention is directed to an antiviral composition against coronavirus, and more specifically an antiviral composition against coronavirus containing an oyster shell powder; an *Inonotus obliquus* powder; and a silkworm powder or a *Gryllus bimaculatus* powder; as an active ingredients.

During research to develop a novel antiviral agent with excellent antiviral activity, the present inventors identified that a composition containing an oyster shell powder; an *Inonotus obliquus* powder; and a silkworm powder or a *Gryllus bimaculatus* powder; as an active ingredients exhibits excellent immune-enhancing activity and inhibitory activity against viral proliferation.

In this regard, in an embodiment of the present invention, macrophages treated with the composition containing the oyster shell powder; the *Inonotus obliquus* powder; and the silkworm powder or the *Gryllus bimaculatus* powder; as an active ingredients were found to significantly increase the production of nitric oxide (NO), IL-6 and TNF-$\alpha$, which induce immune-enhancing activity, compared to a control group not treated with the composition and macrophages treated with a composition excluding any one of the ingredients.

Macrophages are known to be involved in various responses to hosts such as intrinsic immunity and adaptive immunity, to be involved in host defense and homeostasis maintenance, and to produce cytokines such as interleukin-1$\beta$ (IL-1$\beta$), interleukin-6 (IL-6) and tumor necrosis factor-$\alpha$ (TNF-$\alpha$) during the immune response, and thereby play a pivotal role in biodefense in the early stage of infection. In addition, it has been reported that nitric oxide, which is secreted when macrophages respond to substances, has an immunity-enhancing effect.

Therefore, based on these results, the present inventors found that the antiviral composition of the present invention can promote the production of substances having immunity-enhancing activity in macrophages, and thus improves the internal immune system and thus induces effective defensive action from viral infection when infected with harmful substances such as viruses.

In addition, in another embodiment of the present invention, the ability of the antiviral composition of the present invention to inhibit viral proliferation was analyzed. The result showed that a group treated with the composition containing the oyster shell powder; the *Inonotus obliquus* powder; and the silkworm powder or the *Gryllus bimaculatus* powder; was found to have much better ability to inhibit viral proliferation compared to a control group not treated with the composition and a group treated with a composition excluding any one of the ingredients.

Therefore, it was found that the antiviral composition of the present invention can effectively inhibit the proliferation of viruses.

Meanwhile, the antiviral composition according to the present invention contains an oyster shell powder; an *Inonotus obliquus* powder; and a silkworm powder or a *Gryllus bimaculatus* powder; as an active ingredients. Each active ingredient may be present as an ultrafine powder.

Specifically, the powder is provided in the form of a powder of ultrafine particles having a particle size of 50 nm to 5 $\mu$m, and can be quickly and effectively ingested and absorbed by the body, and thereby can rapidly and effectively exhibit antiviral activity.

The oyster shell contained in the antiviral composition of the present invention contains about 95% of calcium carbonate ($CaCO_3$) as a main ingredient, and small amounts of magnesium carbonate ($MgCO_3$) and calcium sulfate ($CaSO_4$). Recent research shows that oyster shell contains large amounts of taurine and betaine as well. Meanwhile, oyster shell is a resource that is mostly wasted in the oyster industry, but in the present invention, components beneficial for the human body contained in oyster shell are recycled, and the utility of oyster shell in applications for antiviral activity is identified.

The oyster shell powder contained in the composition of the present invention may be added in an amount of 40 to 60 parts by weight. When the oyster shell powder is present in an amount less than 40 parts by weight, immunity enhancement and antiviral activity may be insufficient, and when the oyster shell powder is present in an amount higher than 60 parts by weight, the effect is not increased in proportion with the amount of oyster shell powder that is added, and thus economic efficiency is disadvantageously low.

In addition, Chaga (*Inonotus obliquus*) belongs to the Hymenochaetaceae of Basidiomycetes, is a mushroom native to birch trees in Russia, Korea, Eastern Europe and the northern United States, and has been traditionally used for the treatment of cancer, gastritis, ulcers, tuberculosis and the like since the 16th century due to the large amounts of ingredients beneficial for the human body contained therein.

The *Inonotus obliquus* powder contained in the composition of the present invention may be present in an amount of 10 to 30 parts by weight. When the *Inonotus obliquus* powder is added in an amount less than 10 parts by weight, immunity enhancement and antiviral activity may be insufficient, and when the *Inonotus obliquus* powder is added in an amount more than 30 parts by weight, there is a problem in that the acceptability thereof is lowered due to the unique flavor thereof.

In addition, the silkworm is a larva of the silkworm moth, which belongs to the family Lepidoptera, which is known to have the activity of lowering blood sugar by delaying the digestion of sugars and absorption of glucose in the intestine by inhibiting the process of decomposition of disaccharides into monosaccharides in the chorionic membrane of the small intestine and to have useful physiological activities in the body, such as alleviating chronic fatigue, improving memory, alleviating hypertension, improving liver functions and promoting the synthesis of collagen.

The silkworm powder contained in the composition of the present invention may be present in an amount of 10 to 30 parts by weight, and when the silkworm powder is added in an amount less than 10 parts by weight, immunity enhancement and antiviral activity may be insufficient, and when the silkworm powder is present in an amount of 30 parts by weight, there is a problem in that excessive addition of silkworms may cause discomfort.

In addition, *Gryllus bimaculatus,* which is an insect that belongs to the order Orthoptera and the family Gryllidae, and is commonly called "two-spotted cricket" because it has an entirely black body and has a yellow dot at the base of the forewing, the powder of *Gryllus bimaculatus* is known to contain large amounts of unsaturated fatty acids and thus to be capable of alleviating weight gain and reducing the storage of body fats and thereby to have the effect of alleviating weight gain and controlling weight of companion animals, and to contain large amounts of proteins and essential nutrients, and thus has an effect of enhancing immunity.

The *Gryllus bimaculatus* powder contained in the composition of the present invention may be present in an amount of 10 to 30 parts by weight. When the *Gryllus bimaculatus* powder is added in an amount less than 10 parts by weight, immunity enhancement and antiviral activity may be insufficient, and when the *Gryllus bimaculatus* powder is added in an amount more than 30 parts by weight, there is a problem in that excessive addition of silkworms may cause discomfort. Furthermore, the antiviral composition of the present invention may further include a red ginseng powder and the red ginseng powder may be added in an amount of 10 to 20 parts by weight.

The red ginseng is often used as a major raw material for health functional foods due to the effects of facilitating recovery from fatigue and improving physical strength, the effects of improving blood circulation by absorbing excess cholesterol and saturated fats present in the blood in the body, and the effects of improving brain development, treating and preventing hypertension and hypotension, and preventing arteriosclerosis and hyperlipidemia by increasing the flow of blood to the brain.

The red ginseng that may be contained in the composition of the present invention may be present in the form of a powder of ultrafine particulates having a particle size of 50 nm to 5 μm, like the oyster shell powder, the *Inonotus obliquus* powder, the silkworm powder, or the *Gryllus bimaculatus* powder, and may be present in an amount of 10 to 20 parts by weight.

When the red ginseng powder is present in an amount of less than 10 parts by weight, immunity enhancement and antiviral activity may be insufficient, and when the red ginseng powder is present in an amount exceeding 20 parts by weight, the effect is not increased in proportion with the amount of oyster shell powder that is added, and thus economic efficiency is disadvantageously low.

Furthermore, the antiviral composition of the present invention has antiviral activity against coronavirus and variant viruses thereof, and has an activity of effectively inhibiting the proliferation of viruses.

The coronavirus may be a novel coronavirus 19 (COVID-19), a MERS coronavirus (MERS-CoV), a SARS coronavirus (SARS-CoV), or a variant virus thereof, but is not limited thereto, and is preferably a novel coronavirus 19 (COVID-19).

In addition, the antiviral composition according to the present invention may be used as a pharmaceutical composition for preventing or treating diseases related to coronavirus infection.

When the composition of the present invention is used as a pharmaceutical composition, the pharmaceutical composition of the present invention may be formulated and administered in any of various oral or parenteral dosage forms, but is not limited thereto.

Preferably, the composition may be formulated for oral administration, and formulations for oral administration include, for example, tablets, pills, hard/soft capsules, liquids/solutions, suspensions, emulsifiers, syrups, granules, elixirs and the like. In addition to the active ingredients, these formulations may contain diluents (e.g. lactose, dextrose, sucrose, mannitol, sorbitol, cellulose and/or glycine), lubricants (e.g. silica, talc, stearic acid, and magnesium or calcium stearate and/or polyethylene glycol). Tablets may also contain binders such as magnesium aluminum silicate, starch paste, gelatin, methylcellulose, sodium carboxymethylcellulose and/or polyvinylpyrrolidine, and may optionally contain disintegrants such as starch, agar, alginic acid or sodium alginate, or boiling mixtures and/or absorbents, colorants, flavors and sweeteners.

In addition, the amount of the composition of the present invention that is administered to a human may vary depending on the patient's age, weight, gender, dosage form, health condition, and severity of disease. The amount is typically 0.001 to 1,000 mg/day, preferably 0.01 to 500 mg/day, for an adult patient weighing 60 kg, and the composition may be administered once a day or several times a day at regular intervals according to the prescription of a doctor or pharmacist.

In addition, the antiviral composition of the present invention contains natural raw materials as main ingredients and thus is stable in the body and does not cause side effects, and can be used as a food composition for preventing or ameliorating diseases related to coronavirus infection.

In the present invention, the term "food composition" refers to a food that acts advantageously on one or more functions of a subject and improves a state of health, regardless of nutrients fed to the subject ingesting the same. As a result, the food composition may be used for the prevention, amelioration or treatment of diseases or disease-causing factors.

Therefore, as used herein, the term "food composition" may be used as a synonym for "functional food" or "food for a specific nutritional application or pharmaceutical food".

In addition to the antiviral composition of the present invention, the food composition of the present invention may further contain an ordinary pharmaceutically acceptable carrier or excipient, and may be prepared by formulation with a pharmaceutically commonly used additive such as a binder, a disintegrant, a coating agent or a lubricant.

In addition to the active ingredients of the present invention, the food composition of the present invention may contain an additional ingredient, such as any of various flavoring agents or natural carbohydrates, like a conventional food composition.

Examples of the aforementioned natural carbohydrates include monosaccharides, for example, glucose, fructose, and the like, disaccharides, for example, maltose, sucrose and the like, polysaccharides, for example, common sugars such as dextrin and cyclodextrin, and sugar alcohols such as xylitol, sorbitol, and erythritol. The flavoring agent may be advantageously used as a natural flavoring agent (thaumatin), a stevia extract (e.g., rebaudioside A, glycyrrhizin, etc.), and a synthetic flavoring agent (saccharin, aspartame, etc.).

In addition, the food composition of the present invention may be preferably formulated as a food composition containing at least one cytologically or pharmaceutically acceptable carrier, in addition to the active ingredients of the present invention.

The formulation form of the food composition may be a tablet, capsule, powder, granule, liquid, pill, solution, syrup, juice, suspension, emulsion, drop or the like. For example, for formulation in the form of tablets or capsules, the active ingredients may be combined with an oral, nontoxic pharmaceutically acceptable inert carrier such as ethanol, glycerol or water. In addition, if desired or necessary, a suitable binder, lubricant, disintegrant or coloring agent may also be contained as a mixture. Examples of suitable binders include, but are not limited to, natural sugars such as starch, gelatin, glucose or beta-lactose, corn sweeteners, natural and synthetic gums such as acacia, lacquercanth or sodium oleate, sodium stearate, magnesium stearate, sodium benzoate, sodium acetate, sodium chloride, and the like. Disintegrants include, but are not limited to, starch, methyl cellulose, agar, bentonite, xanthan gum, and the like. The pharmaceutically acceptable carrier in the composition formulated as a liquid solution may be saline, sterile water, Ringer's solution, buffered saline, albumin injection solution, dextrose solution, maltodextrin solution, glycerol or ethanol, each of which is sterile and biocompatible, and a combination of one or more of these components, and other conventional additives such as antioxidants, buffers or bacteriostatic agents may be added if necessary. In addition, injectable formulations such as aqueous solutions, suspensions, emulsions, and pills, capsules, granules, or tablets may be prepared by further adding diluents, dispersants, surfactants, binders and lubricants.

The food composition of the present invention formulated in the above manner may be used as a functional food, or may be added to various foods.

Examples of the food to which the composition of the present invention can be added include beverages, meat, chocolate, foods, confectionery, pizza, ramen, other noodles, gums, candy, ice cream, alcoholic beverages, vitamin complexes, and health supplements.

In addition, in addition to the active ingredients of the present invention, the food composition may contain any of various nutrients, vitamins, minerals (electrolytes), flavoring agents such as synthetic flavoring agents and natural flavoring agents, coloring agents, fillers (cheese, chocolate, etc.), pectic acid and salts thereof, alginic acid and salts thereof, organic acids, protective colloidal thickeners, pH adjusters, stabilizers, preservatives, glycerin, alcohols, carbonates used in carbonated beverages, and the like. In addition, the food composition of the present invention may contain flesh for the preparation of natural fruit juice, fruit juice beverages and vegetable beverages.

Accordingly, the present invention provides a health functional food for preventing or ameliorating viral infectious diseases containing an oyster shell powder; an *Inonotus obliquus* powder; and a silkworm powder or a *Gryllus bimaculatus* powder; as an active ingredients.

The health functional food of the present invention can be prepared and processed into a formulation such as a tablet, capsule, powder, granule, liquid or pill for preventing or improving diseases caused by infection with a virus, preferably coronavirus.

As used herein, the term "health functional food" refers to a food that is prepared and processed using raw materials or ingredients having functions useful for humans according to the Health Functional Food Acts, and means a food that is ingested in order to control nutrients associated with the organization and functions of the human body and obtain effects useful for health-related applications such as physiological action.

The health functional food of the present invention may contain a typical food additive, and whether or not the food additive is suitable is determined based on the rules and standards on the corresponding item according to general principles and general test methods for Food Additives Codex approved by the Ministry of Food and Drug Safety, unless otherwise specified.

Examples of the items listed in the "Food Additives Codex" include: chemical compounds such as ketones, glycine, calcium citrate, nicotinic acid, and cinnamic acid, natural additives such as Persimmon color, licorice extract, crystalline cellulose, Kaoliang color and guar gum, mixed preparations, such as a sodium L-glutamate preparation, an alkali additive for noodles, a preservative preparation, a tar color preparation and the like.

For example, the tablet-type health functional food may be obtained by granulating a mixture of the active ingredients of the present invention with an excipient, a binder, a disintegrant and other additives using a conventional method and then compression-molding the resulting product with a lubricant, or by directly compression-molding the resulting mixture. In addition, the tablet-type health functional food may contain a flavor enhancer or the like, if necessary.

Among capsule-type health functional foods, the hard capsule-type health functional food may be prepared by filling a conventional hard capsule with the active ingredients of the present invention, and the soft capsule-type health functional food may be prepared by filling a capsule base such as gelatin with a mixture of the extract and additives such as excipients. The soft capsule may contain a plasticizer such as glycerin or sorbitol, a colorant or a preservative, if necessary.

The pill-type health functional food may be prepared by molding a mixture of the active ingredients of the present invention with excipients, binders, disintegrants or the like by a conventionally known method, and may be coated with white sugar or other coating agent, if necessary. Alternatively, the pill-type health functional food may be surface-coated with a material such as starch or talc.

The granule-type health functional food may be prepared in the form of a granule from a mixture of the active ingredients of the present invention with excipients, binders, disintegrants and the like by a conventionally known method, and may contain a flavoring agent, a flavor enhancer or the like, if necessary.

The health functional food may be a beverage, meat, chocolate, food, confectionery, pizza, ramen, other type of noodle, gum, candy, ice cream, alcoholic beverage, vitamin complex, health supplement or the like.

Hereinafter, the present invention will be described in more detail with reference to examples. The examples are provided only for illustration of the present invention and should not be construed as limiting the scope of the present invention.

**<Example 1>**

**Preparation of antiviral powder composition**

A composition having antiviral activity was prepared in the following manner. First, as raw materials, oyster shells, *Inonotus obliquus*, silkworms and *Gryllus bimaculatus* were obtained from commercially available products. The materials were washed thoroughly with water and dried, and then each of the materials was pulverized in the form of a powder of ultrafine particles having a size of 50 nm to 5 $\mu$m using a dry nano grinder employing a top-down method. Then, the powders were mixed in the amounts shown in Table 1 to prepare a mixed powder composition. At this time, red ginseng having an immune-enhancing function was further prepared as a powder of ultra-fine particles in the same manner as described above, and was then used to prepare a mixed powder composition as shown in Table 1 below.

[Table 1]

| Ingredient | Preparation Example 1 | Preparation Example 2 | Preparation Example 3 | Preparation Example 4 | Comparative Example 1 | Comparative Example 2 | Comparative Example 3 |
|---|---|---|---|---|---|---|---|
| | | | Antiviral composition | | | | |
| Ultrafine oyster shell powder | 50g | 50g | 50g | 50g | - | 50g | 50g |
| Ultrafine *Inonotus obliquus* powder | 30g | 30g | 20g | 20g | 30g | - | 20g |
| Ultrafine silkworm powder | 20g | - | 15g | 10g | 20g | 20g | - |
| Ultrafine *Gryllus bimaculatus* powder | - | 20g | 15g | 10g | 20g | 20g | - |
| Ultrafine red ginseng powder | - | - | - | 10g | 20g | 20g | 20g |

The mixed powder obtained by mixing the ultrafine powders was packaged in an individual unit to prepare the antiviral powder composition of the present invention. More specifically, as shown in FIG. 1, all of the raw materials were added and then thoroughly mixed while stirring at a temperature of 20°C for 10 minutes. Then, nano-powderization was performed for 15 minutes to obtain a nanoparticle size of 50 nm to 5 $\mu$m. Then, electric charges were generated using an ebonite charger at a temperature of 25°C for 10 seconds. Then, the particle size was classified depending on charge deviation, and the charge was dissociated at a temperature of 25°C for 10 seconds. After zinc activation, the resulting product was weighed, placed in a certain amount into capsules and then packaged.

**<Example 2>**

**Analysis of antiviral activity of composition of present invention**

Whether or not the antiviral solution prepared by dissolving each of the antiviral mixture powders prepared in Example 1 in 100 ml of distilled water had virus inhibitory activity was evaluated in the following manner.

<2-1> Immune-enhancing activity assay

The body immune system, which is a defense system against bacteria and viruses, is the forefront system that acts as a defense against a disease. When immunity is lowered, the viral infection defense system does not function properly, thus increasing vulnerability to virus infection.

Accordingly, the present inventors performed an experiment to determine whether or not the antiviral composition of Example 1 has immune-enhancing activity. For this purpose, the amount of nitric oxide produced in macrophages was analyzed. Macrophages are cells that play an important role in innate immunity, and a factor that is distributed in all tissues in the animal body, is responsible for the innate immune response, and plays an important role in the immunity activation of macrophages is nitric oxide (NO).

Therefore, a macrophage cell line (RAW 264.7) was treated with the composition (200 ug/ml) of the present invention, and the amount of NO produced in macrophages was measured. The amount of NO produced was measured using the Griess reaction of $NO_2$-(nitrite), which is a stable nitric oxide-oxide.

[Table 2]

| | Preparation Example 1 | Preparation Example 2 | Preparation Example 3 | Preparation Example 4 | Comparative Example 1 | Comparative Example 2 | Comparative Example 3 |
|---|---|---|---|---|---|---|---|
| Measurement of amount of NO produced | | | | | | | |
| $NO_2$ content (uM) | 3.7 | 3.8 | 4.1 | 4.5 | 1.5 | 2.1 | 2.2 |

As a result, as shown in Table 2, the compositions of Preparation Examples 1 to 4 according to the present invention were found to have a higher amount of nitric oxide produced in macrophages than the compositions of Comparative Examples, and in particular, the composition containing all of oyster shells, *Inonotus obliquus,* silkworms and *Gryllus bimaculatus* was found to have the highest nitric oxide-producing activity, which means that the composition has the highest immunity-enhancing effect.

Furthermore, the present inventors measured the levels of production of TNF-a and IL-4, which are cytokines acting on immunity enhancement, in the experimental groups. Measurement of these cytokines was carried out using ELISA.

The result showed that the experimental groups of Preparation Examples 1 to 4, which are compositions of the present invention, increased the production of TNF-a and IL-4 compared to the Comparative Examples, similar to nitric oxide, and the composition of Preparation Example 4 containing all of oyster shells, *Inonotus obliquus,* silkworms and *Gryllus bimaculatus* was found to have the highest production levels.

<2-2> Antiviral activity assay

The following experiment was performed to determine whether or not the antiviral composition of the present invention has antiviral activity.

For this purpose, $2 \times 10^4$ Vero cells were seeded in each well of a 96-well plate and cultured for 24 hours. After 24 hours, the culture supernatant of each well was removed, 90 μl of the virus solution diluted to TCID50 was added to each well, 10 μl of each composition prepared in Example 1 was administered to each well, and the composition was cultured for 24 hours. The virus used in the experiment was a Corona 19 virus (NCCP 43326 Human corona virus, BetaCoV/Korea/KCDC03/2020) isolated in Korea, which was received from the Korea Centers for Disease Control and Prevention (KCDC), and experiments were conducted under BL (Biosafety Level) 3.

Then, the viral proliferation inhibitory activity of each well treated with the composition was calculated using the following equation. The viral proliferation inhibitory activity used in this experiment was determined by the method disclosed in Korean Patent No. 10-0682069.

$$\text{Viral proliferation inhibitory activity (\%)} = \frac{D - C}{B - C} \times 100$$

B: Absorbance (560 nm) in cells not infected with virus

C: Absorbance (560 nm) in cells infected with virus

D: Absorbance (560 nm) in cells administered with both antiviral composition and virus

[Table 3]

| Result of analysis of antiviral activity (viral inhibition (%)) | | |
|---|---|---|
| Item | Virus infection group | |
| (Proliferation inhibition %) | Group not treated with antiviral composition | Group treated with antiviral composition |
| Preparation Example 1 | 0.01 ± 10.40 | 82.02 ± 0.78 |
| Preparation Example 2 | 0.00 ± 11.60 | 84.03 ± 0.99 |

(continued)

| Result of analysis of antiviral activity (viral inhibition (%)) | | |
|---|---|---|
| Item | Virus infection group | |
| (Proliferation inhibition %) | Group not treated with antiviral composition | Group treated with antiviral composition |
| Preparation Example 3 | 0.00 ± 12.44 | 90.01 ± 0.89 |
| Preparation Example 4 | 0.00 ± 11.40 | 95.05 ± 0.99 |
| Comparative Example 1 | 0.02 ± 08.54 | 22.10 ± 0.71 |
| Comparative Example 2 | 0.01 ± 10.12 | 32.05 ± 0.99 |
| Comparative Example 3 | 0.00 ± 11.63 | 35.01 ± 0.98 |

As shown in Table 3, the result shows that the compositions of Preparation Examples 1 to 4, which are antiviral compositions of the present invention, were found to have better inhibitory activity against proliferation of the Corona 19 virus, compared to the compositions of Comparative Examples. In particular, the composition of Preparation Example 4, containing all of oyster shells, *Inonotus obliquus,* silkworms and *Gryllus bimaculatus,* was found to have proliferation inhibitory ability of 95% or more against the Corona 19 virus, which indicates that the composition of Preparation Example 4 can be used as an excellent antiviral composition.

As is apparent from the foregoing, the present invention relates to an antiviral composition containing an oyster shell powder; an *Inonotus obliquus* powder; and a silkworm powder or a *Gryllus bimaculatus* powder; as an active ingredients. The antiviral composition according to the present invention has activity of enhancing immunity that acts as an *in-vivo* defense against infection with harmful substances such as bacteria and viruses, and has activity of effectively inhibiting the proliferation of coronavirus, and has thus useful effects as a drug or health functional food for the prevention, amelioration or treatment of coronaviral infection.

Although the preferred embodiments of the present invention have been disclosed, those skilled in the art will appreciate that various modifications, additions and substitutions are possible, without departing from the scope and spirit of the invention as disclosed in the accompanying claims. Therefore, the disclosed embodiments should be considered from an illustrative point of view rather than a limiting point of view. The scope of the present invention is defined by the claims rather than the aforementioned description, and all differences within the scope of equivalent thereto should be construed as falling into the scope of the present invention.

## Claims

1.  An antiviral composition comprising
    an oyster shell powder;
    an *Inonotus obliquus* powder; and
    a silkworm powder or a *Gryllus bimaculatus* powder as an active ingredients.

2.  The antiviral composition according to claim 1, wherein the oyster shell powder is present in an amount of 40 to 60 parts by weight, the *Inonotus obliquus* powder is present in an amount of 10 to 30 parts by weight, and the silkworm powder or the *Gryllus bimaculatus* powder is present in an amount of 10 to 30 parts by weight.

3.  The antiviral composition according to claim 1, further comprising a red ginseng powder,
    wherein the red ginseng powder is present in an amount of 10 to 20 parts by weight.

4.  The antiviral composition according to claim 1, wherein the powder is a powder of ultrafine particles having a particle size of 50 nm to 5 μm.

5.  The antiviral composition according to claim 1, for use in preventing or treating a coronaviral infection.

Fig.1

```
                                    ┌──────────────────────┐
                                    │    Raw materials     │
                                    └──────────────────────┘
                                             │ (Nano-calcium)
        ┌──────────────────────┐    ┌──────────────────────┐    ┌────────────────────────────┐
        │  Additive injection  │────│       Mixing         │    │  15-30°, 10-15 minutes     │
        └──────────────────────┘    └──────────────────────┘    └────────────────────────────┘
                                             │
                                    ┌──────────────────────┐    ┌────────────────────────────┐
                                    │  Nano-powderization  │    │  (50 nm-5 um )             │
                                    └──────────────────────┘    │  10-15 minutes             │
                                             │                  └────────────────────────────┘
                                    ┌──────────────────────┐    ┌────────────────────────────┐
                                    │   Charge injection   │    │ (Generation of charges     │
                                    └──────────────────────┘    │ using ebonite charger)     │
                                             │                  │ 15-30°C / 5-15 seconds     │
                                    ┌──────────────────────┐    └────────────────────────────┘
                                    │Particle size screening│   ┌────────────────────────────┐
                                    └──────────────────────┘    │ Particle size classification│
                                             │                  │ depending on charge deviation│
                                    ┌──────────────────────┐    └────────────────────────────┘
                                    │  Charge dissociation │    ┌────────────────────────────┐
                                    └──────────────────────┘    │  15-30°C / 5-15 seconds    │
                                             │                  └────────────────────────────┘
                                    ┌──────────────────────┐
                                    │   Zinc activation    │
                                    └──────────────────────┘
                                             │
                                    ┌──────────────────────┐
                                    │      Weighing        │
                                    └──────────────────────┘
                                             │
                                    ┌──────────────────────┐    ┌────────────────────────────┐
                                    │   Capsule filling    │    │   (automated filler)       │
                                    └──────────────────────┘    └────────────────────────────┘
                                             │
                                    ┌──────────────────────┐
                                    │   Bottle packaging   │
                                    └──────────────────────┘
                                             │
                                    ┌──────────────────────┐
                                    │      Shipment        │
                                    └──────────────────────┘
```

Europäisches
Patentamt

European
Patent Office

Office européen
des brevets

# EUROPEAN SEARCH REPORT

Application Number

EP 20 20 5703

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2016 0124408 A (NAT UNIV CHONBUK IND COOP FOUND [KR]) 27 October 2016 (2016-10-27) * claims 1-6 * | 1-5 | INV. A61K35/618 A61K35/64 A61K36/07 A61P31/12 A61P31/14 |
| X | CN 111 265 635 A (YANG DESHENG; YANG JINGQUN) 12 June 2020 (2020-06-12) * claims 1-3 * | 1-5 | |
| X | XIONG XINGJIANG ET AL: "Chinese herbal medicine for coronavirus disease 2019: A systematic review and meta-analysis", PHARMACOLOGICAL RESEARCH, ACADEMIC PRESS, LONDON, GB, vol. 160, 2 July 2020 (2020-07-02), XP086320456, ISSN: 1043-6618, DOI: 10.1016/J.PHRS.2020.105056 [retrieved on 2020-07-02] * table 1 * | 1-5 | |
| X | KR 2020 0099114 A (KWON DUR HAN [KR]) 21 August 2020 (2020-08-21) * the whole document * | 1-5 | TECHNICAL FIELDS SEARCHED (IPC) A61K A61P |
| X | KR 2020 0071573 A (GYEONGGIDO BUSINESS & SCIENCE ACCELERATOR [KR]) 19 June 2020 (2020-06-19) * claims 1-4 * | 1-5 | |
| X | KR 2020 0071572 A (GYEONGGIDO BUSINESS & SCIENCE ACCELERATOR [KR]) 19 June 2020 (2020-06-19) * the whole document * | 1-5 | |

The present search report has been drawn up for all claims

| Place of search | Date of completion of the search | Examiner |
|---|---|---|
| Munich | 17 April 2021 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 960 189 A1**

## ANNEX TO THE EUROPEAN SEARCH REPORT
## ON EUROPEAN PATENT APPLICATION NO.

EP 20 20 5703

17-04-2021

| Patent document cited in search report | Publication date | Patent family member(s) | Publication date |
|---|---|---|---|
| KR 20160124408 A | 27-10-2016 | NONE | |
| CN 111265635 A | 12-06-2020 | NONE | |
| KR 20200099114 A | 21-08-2020 | NONE | |
| KR 20200071573 A | 19-06-2020 | NONE | |
| KR 20200071572 A | 19-06-2020 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101976528
- KR 102109196
- KR 100682069